# EUROPEAN PATENT APPLICATION

(11) **EP 4 454 593 A1**
(43) Date of publication of application: **30.10.2024**
(21) Application number: 22911228.9
(22) Date of filing: 20.12.2022
(51) Int. Cl.: A61B 34/35, B25J 3/00

(54) **SURGERY ASSISTING SYSTEM AND SURGERY ASSISTING ROBOT**

(30) Priority: 22.12.2021 JP 2021208662
(71) Applicant: KAWASAKI JUKOGYO KABUSHIKI KAISHA, Kobe-shi, Hyogo 650-8670 (JP)
(72) Inventor: TOJO, Tsuyoshi, Hyogo 650-8670 (JP); KUNO, Hirotaka, Hyogo 650-8670 (JP); YASUDA, Tatsurou, Hyogo 650-8670 (JP)
(74) Representative: Hoffmann Eitle
(86) International application number: PCT/JP2022/046911
(87) International publication number: WO 2023/120526

(57) **Abstract**

A robotic surgical system (100) includes a board (140) in a robot arm (60) or an arm operation unit (80), to which a signal received by the arm operation unit (80) is input, and a controller (31a). The controller (31a) is connected to the board (140) by serial communication via a first wire line (141).

## Description

### Technical Field

The present disclosure relates to a robotic surgical system and a surgical robot.

### Background Art

Conventionally, a robotic system is disclosed. Japanese Patent Laid-Open No. 2019-162427 discloses a robotic system including a surgical instrument and an arm. The surgical instrument is arranged at a distal end of the arm. An operation unit is spaced apart from the arm. A joystick and an operation button are arranged on the operation unit. The arm moves when an operator operates the operation unit. Usually, wire lines that transmit signals from the operation unit are connected to a controller that controls the robotic system. The number of wire lines required is equal to the number of joysticks and operation buttons.

### Prior Art

### Patent Document

Patent Document 1: Japanese Patent Laid-Open No. 2019-162427

### Summary of the Invention

When the number of wire lines extending from the operation unit to the controller is equal to the number of joysticks and operation buttons, as described above, the wire lines from the operation unit to the controller become thick and heavy, and the bending load increases. Thus, in particular, when the operation unit is placed at the distal end of the robot arm, both a structure and a drive must be large, which affects miniaturization. Therefore, it is desired to reduce the number of wire lines extending to the controller.

The present disclosure is intended to solve the above problem. The present disclosure aims to provide a robotic surgical system and a surgical robot each capable of reducing the number of wire lines extending to a controller.

A robotic surgical system according to a first aspect of the present disclosure includes a robot arm to which a surgical instrument is attached, an arm operation unit attached to the robot arm to operate the robot arm, a board provided in the robot arm or the arm operation unit and to which a signal received by the arm operation unit is input, and a controller. The controller is connected to the board by serial communication via a first wire line.

In the robotic surgical system according to the first aspect of the present disclosure, as described above, the board to which the signal received by the arm operation unit is input is connected to the controller by serial communication via the first wire line. Accordingly, even when a joystick, an operation button, etc. are arranged on the arm operation unit, wire lines extending from the joystick, the operation button, etc. are connected to the board, while the board is connected to the controller by serial communication. Consequently, the number of wire lines can be reduced as compared with a case in which the joystick, the operation button, etc. are each connected to the controller.

A surgical robot according to a second aspect of the present disclosure includes a robot arm to which a surgical instrument is attached, an arm operation unit attached to the robot arm to operate the robot arm, a board provided in the robot arm or the arm operation unit and to which a signal received by the arm operation unit is input, and a controller. The controller is connected to the board by serial communication via a wire line.

In the surgical robot according to the second aspect of the present disclosure, as described above, the board to which the signal received by the arm operation unit is input is connected to the controller by serial communication via the wire line. Accordingly, even when a joystick, an operation button, etc. are arranged on the arm operation unit, wire lines extending from the joystick, the operation button, etc. are connected to the board, while the board is connected to the controller by serial communication. Consequently, it is possible to provide the surgical robot capable of reducing the number of wire lines as compared with a case in which the joystick, the operation button, etc. are each connected to the controller.

According to the present disclosure, the number of wire lines extending to the controller can be reduced.

### Brief Description of the Drawings

FIG. 1 is a diagram showing the configuration of a robotic surgical system according to an embodiment.
FIG. 2 is a diagram showing the configuration of a surgical robot according to the embodiment.
FIG. 3 is a diagram showing the configuration of a robot arm according to the embodiment.
FIG. 4 is a perspective view showing the configuration of an arm operation unit according to the embodiment.
FIG. 5 is a diagram showing an endoscope.
FIG. 6 is a diagram showing a pivot position setting instrument.
FIG. 7 is a diagram for illustrating translational movement of the robot arm.
FIG. 8 is a diagram for illustrating rotational movement of the robot arm.
FIG. 9 is an exploded perspective view of the robot arm with an adapter and a medical instrument removed therefrom, according to the embodiment.
FIG. 10 is a perspective view of the adapter and the surgical instrument according to the embodiment, as viewed from the Y2 direction side.
FIG. 11 is a control block diagram of the surgical robot according to the embodiment.
FIG. 12 is a control block diagram of the robot arm according to the embodiment.
FIG. 13 is a diagram showing a serial connection between a controller and a board according to the embodiment.
FIG. 14 is a sectional view taken along the line 300-300 in FIG. 4.

### Modes for Carrying Out the Invention

### Configuration of Robotic Surgical System

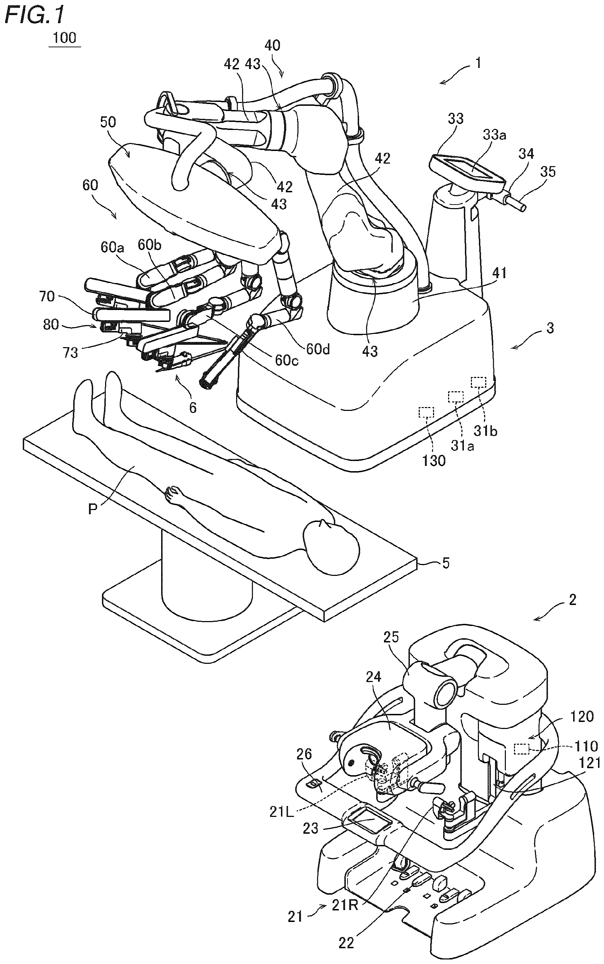

The configuration of a robotic surgical system 100 according to the present embodiment is now described with reference to FIGS. 1 to 14. The robotic surgical system 100 includes a surgical robot 1 and a remote control apparatus 2.

The surgical robot 1 is a patient P-side apparatus. The surgical robot 1 includes a medical cart 3, and is movable. The surgical robot 1 is arranged in an operating room. The remote control apparatus 2 is an operator-side apparatus for operating the surgical robot 1. The remote control apparatus 2 is spaced apart from the surgical robot 1, and the surgical robot 1 is remotely controlled by the remote control apparatus 2. An operator such as a doctor inputs a command to the remote control apparatus 2 to cause the surgical robot 1 to perform a desired operation. The remote control apparatus 2 transmits the input command to the surgical robot 1. The surgical robot 1 operates based on the received command. The surgical robot 1 is arranged in the operating room that is a sterilized sterile field.

### Configuration of Surgical Robot

As shown in FIG. 1, the surgical robot 1 includes the medical cart 3, a positioner 40, an arm base 50, a plurality of robot arms 60, and arm operation units 80.

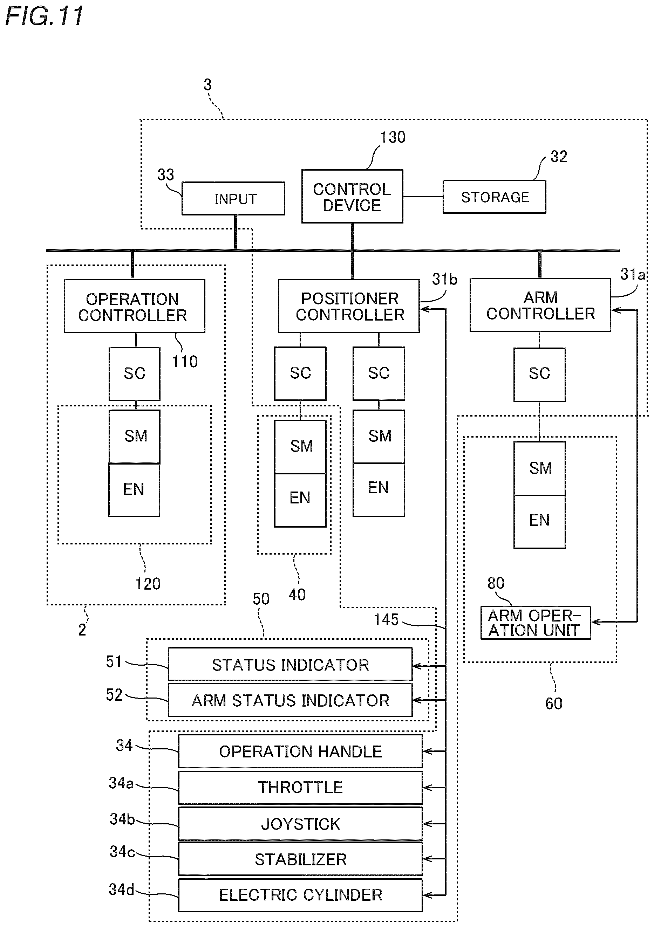

The medical cart 3 moves the positioner 40. The medical cart 3 includes an input 33. The input 33 receives operations to move the positioner 40, the arm base 50, and the plurality of robot arms 60 or change their postures mainly in order to prepare for surgery before the surgery. The input 33 includes a display 33a. The display 33a is a liquid crystal panel, for example. The medical cart 3 includes an operation handle 34, a throttle 34a, a joystick 34b, a stabilizer 34c, and an electric cylinder 34d that are shown in FIG. 11. The operation handle 34 and the throttle 34a receive an operator's steering operation. The joystick 34b receives an operation to move the positioner 40.

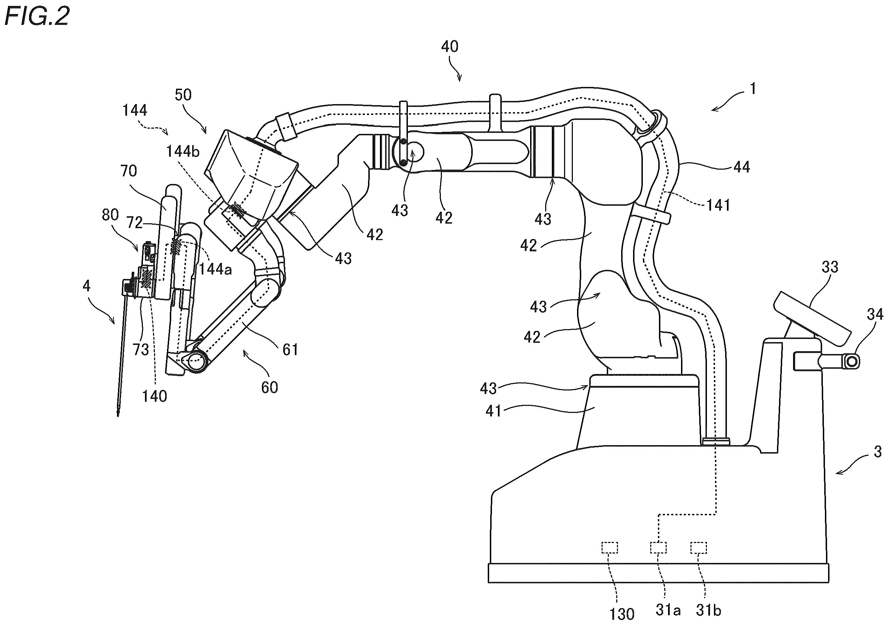

As shown in FIG. 2, the positioner 40 includes a 7-axis articulated robot, for example. The positioner 40 is arranged on the medical cart 3. The positioner 40 adjusts the position of the arm base 50. The positioner 40 moves the position of the arm base 50 three-dimensionally.

The positioner 40 includes a base 41 and a plurality of links 42 coupled to the base 41. The plurality of links 42 are coupled to each other by joints 43.

The arm base 50 is attached to a distal end of the positioner 40. A proximal end of each of the plurality of robot arms 60 is attached to the arm base 50. Each of the plurality of robot arms 60 is able to take a folded and stored posture. The arm base 50 and the plurality of robot arms 60 are covered with sterile drapes and used. Moreover, each of the robot arms 60 supports a surgical instrument 4.

A status indicator 51 and an arm status indicator 52 that are shown in FIG. 11 are provided on the arm base 50. The status indicator 51 indicates the status of the robotic surgical system 100. The arm status indicator 52 indicates the statuses of the robot arms 60.

As shown in FIG. 1, the plurality of robot arms 60 are arranged. Specifically, four robot arms 60a, 60b, 60c, and 60d are arranged. The robot arms 60a, 60b, 60c, and 60d have the same or similar configurations as each other.

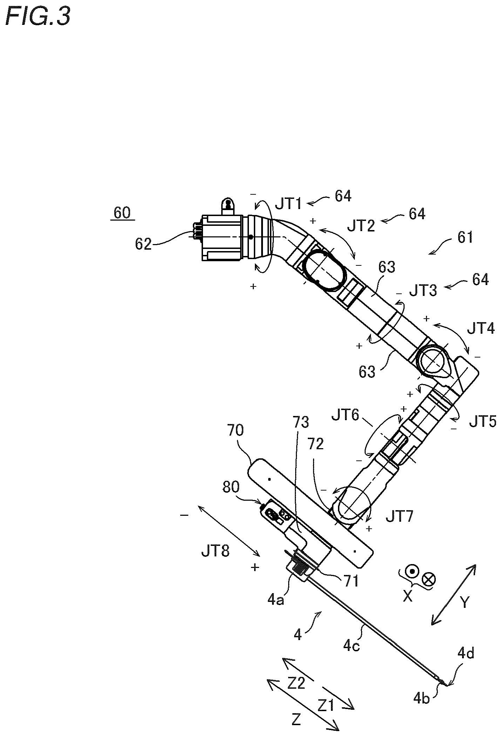

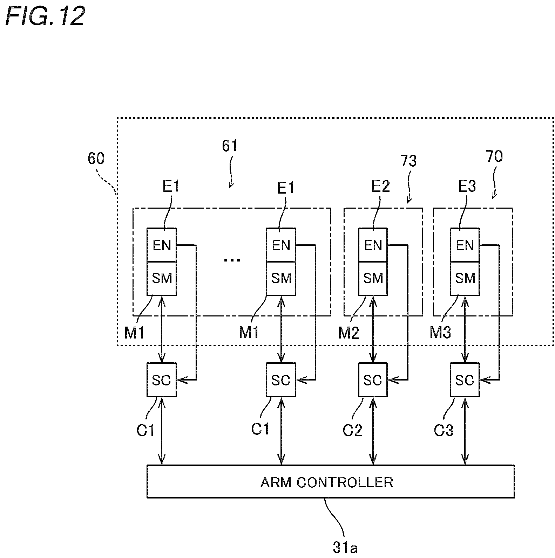

As shown in FIG. 3, each robot arm 60 includes an arm portion 61, a first link 72, a second link 73, and a translation mechanism 70. The robot arm 60 includes JT1 to JT7 axes as rotation axes and a JT8 axis as a linear motion axis. The JT1 to JT7 axes are rotation axes of joints 64 of the arm portion 61. Furthermore, the JT7 axis is a rotation axis of the first link 72. The JT8 axis is a linear motion axis along which the translation mechanism 70 moves the second link 73 relative to the first link 72 along a Z direction. That is, a servomotor M1 shown in FIG. 12 is arranged for each of the JT1 to JT7 axes of the robot arm 60. A servomotor M3 is arranged with respect to the JT8 axis.

The arm portion 61 includes a 7-axis articulated robot arm. The first link 72 is placed at a distal end of the arm portion 61. An arm operation unit 80 described below is attached to the second link 73. The translation mechanism 70 is arranged between the first link 72 and the second link 73. A holder 71 that holds the surgical instrument 4 is arranged on the second link 73.

The surgical instrument 4 is attached to a distal end of each of the plurality of robot arms 60. The surgical instrument 4 includes a replaceable instrument, an endoscope 6 to capture an image of a surgical site, etc. The surgical instrument 4 as the instrument includes a driven unit 4a, a pair of forceps 4b, and a shaft 4c. The driven unit 4a, the shaft 4c, and the pair of forceps 4b are arranged along the Z direction.

As shown in FIG. 1, the endoscope 6 is attached to the distal end of one of the plurality of robot arms 60, such as the robot arm 60c, and surgical instruments 4 other than the endoscope 6 are attached to the distal ends of the remaining robot arms 60a, 60b, and 60d, for example. The endoscope 6 is attached to one of two robot arms 60b and 60c arranged in the center among the four robot arms 60 arranged adjacent to each other.

### Configuration of Instrument

As shown in FIG. 3, the pair of forceps 4b is provided at a distal end of the instrument, for example. At the distal end of the instrument, in addition to the pair of forceps 4b, a pair of scissors, a grasper, a needle holder, a microdissector, a stable applier, a tacker, a suction cleaning tool, a snare wire, a clip applier, etc. are arranged as instruments having joints. At the distal end of the instrument, a cutting blade, a cautery probe, a washer, a catheter, a suction orifice, etc. are arranged as instruments having no joint.

### Configuration of Arm Operation Unit

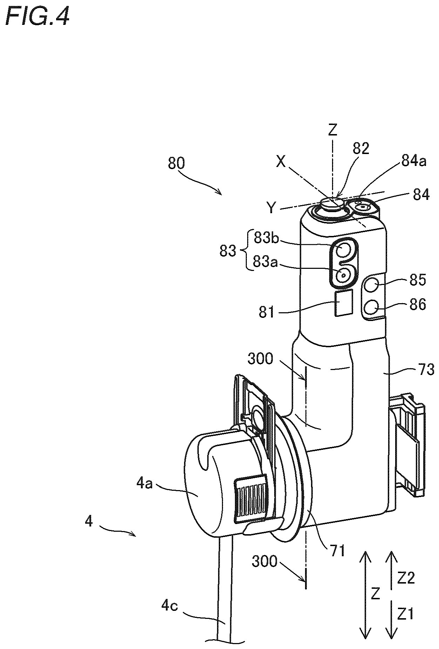

As shown in FIG. 4, the arm operation unit 80 is attached to the robot arm 60 to operate the robot arm 60. Specifically, the arm operation unit 80 is attached to the second link 73.

The arm operation unit 80 includes an enable switch 81, a joystick 82, and linear switches 83, a pivot button 85, an adjustment button 86, a mode switching button 84, and a mode indicator 84a.

The enable switch 81 enables or disables movement of the robot arm 60 in response to the joystick 82 and the linear switches 83. When the enable switch 81 is pressed by an operator such as a nurse or an assistant grasping the arm operation unit 80, movement of the surgical instrument 4 by the robot arm 60 is enabled.

The joystick 82 is an operation tool to control movement of the surgical instrument 4 by the robot arm 60. The joystick 82 controls a moving direction and a moving speed of the robot arm 60. The robot arm 60 is moved in accordance with a tilting direction and a tilting angle of the joystick 82.

The linear switches 83 are switches to control movement of the surgical instrument 4 by the robot arm 60 in a direction along the longitudinal direction of the surgical instrument 4. The linear switches 83 include a linear switch 83a to move the surgical instrument 4 in a direction in which the surgical instrument 4 is inserted into a patient P, and a linear switch 83b to move the surgical instrument 4 in a direction in which the surgical instrument 4 is moved away from the patient P. Both the linear switch 83a and the linear switch 83b are pushbutton switches.

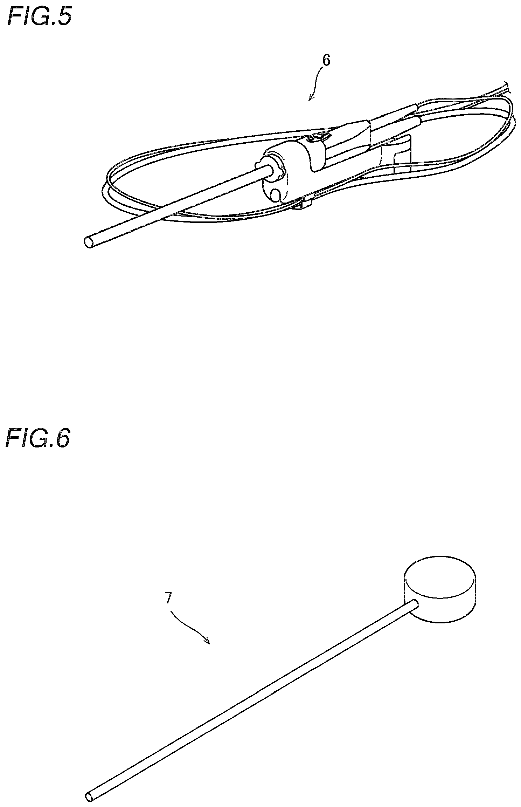

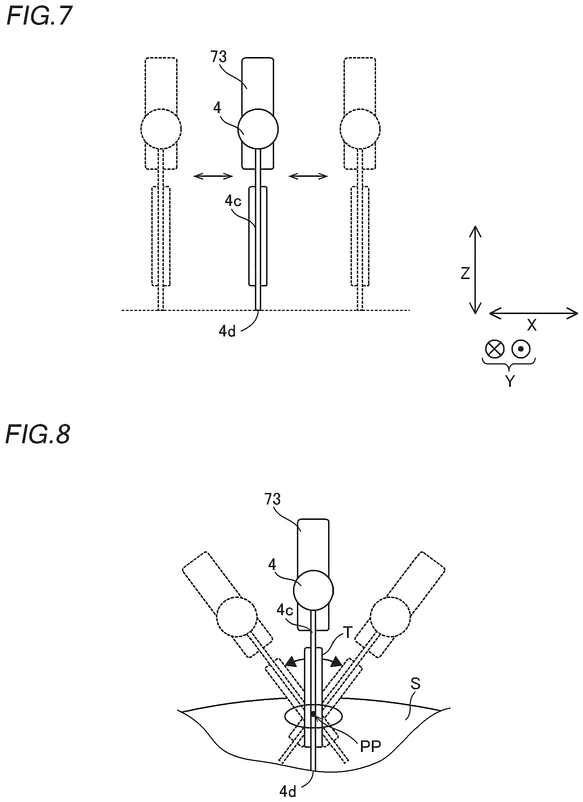

The pivot button 85 is a button to set a pivot position PP that serves as a fulcrum for movement of the surgical instrument 4 attached to the robot arm 60. When the pivot button 85 is pressed in a state in which a distal end of the endoscope 6 shown in FIG. 5 or a pivot position setting instrument 7 shown in FIG. 6 has been moved to a position corresponding to an insertion position of a trocar T inserted into the body surface S of the patient P, which is shown in FIG. 8, the pivot position PP is set and stored in a storage 32 shown in FIG. 11. The pivot position PP is set as one point, and in pivot position PP setting, the direction of the surgical instrument 4 is not set. The pivot position PP is individually set for each of the plurality of robot arms 60.

As shown in FIG. 4, the adjustment button 86 is a button to optimize the position of the robot arm 60. After the pivot position PP for the robot arm 60 to which the endoscope 6 has been attached is set, the positions of the other robot arms 60 and the arm base 50 are optimized when the adjustment button 86 is pressed.

The mode switching button 84 is a button to switch between a mode for translationally moving the surgical instrument 4 as shown in FIG. 7 and a mode for rotationally moving the surgical instrument 4 as shown in FIG. 8. As shown in FIG. 7, in the mode for translationally moving the robot arm 60, the robot arm 60 is moved such that a distal end 4d of the surgical instrument 4 is moved in an X-Y plane. As shown in FIG. 8, in the mode for rotationally moving the robot arm 60, the robot arm 60 is moved such that the surgical instrument 4 is rotationally moved about the pair of forceps 4b when any pivot position PP is not set, and the surgical instrument 4 is rotationally moved about the pivot position PP as a fulcrum when the pivot position PP is set. In this case, the surgical instrument 4 is rotationally moved with the shaft 4c of the surgical instrument 4 inserted into the trocar T. The mode switching button 84 is arranged on a Z-direction side surface of the arm operation unit 80.

The mode indicator 84a indicates a selected mode. The mode indicator 84a is on to indicate a rotational movement mode and is off to indicate a translational movement mode. Furthermore, the mode indicator 84a also serves as a pivot position indicator that indicates that the pivot position PP has been set. The mode indicator 84a is arranged on the Z-direction side surface of the arm operation unit 80.

### Configuration of Surgical Instrument, Adapter, Drape, and Arm

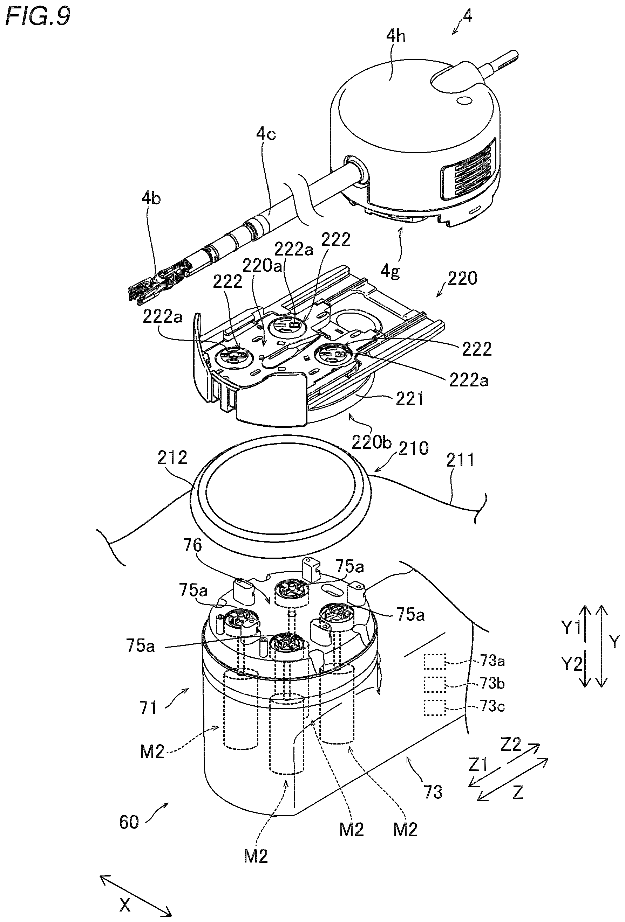

As shown in FIG. 9, the surgical instrument 4 is removably connected to the robot arm 60 via an adapter 220. The adapter 220 is arranged between servomotors M2 of the robot arm 60 and the surgical instrument 4. The adapter 220 is a drape adapter for holding a drape 210, and is replaced by a user every time a surgery is performed. Thus, the drape 210 can be held using the adapter 220. The drape 210 is a drape for covering the robot arm 60 and is sterilized. The drape 210 is sandwiched between the adapter 220 and the robot arm 60.

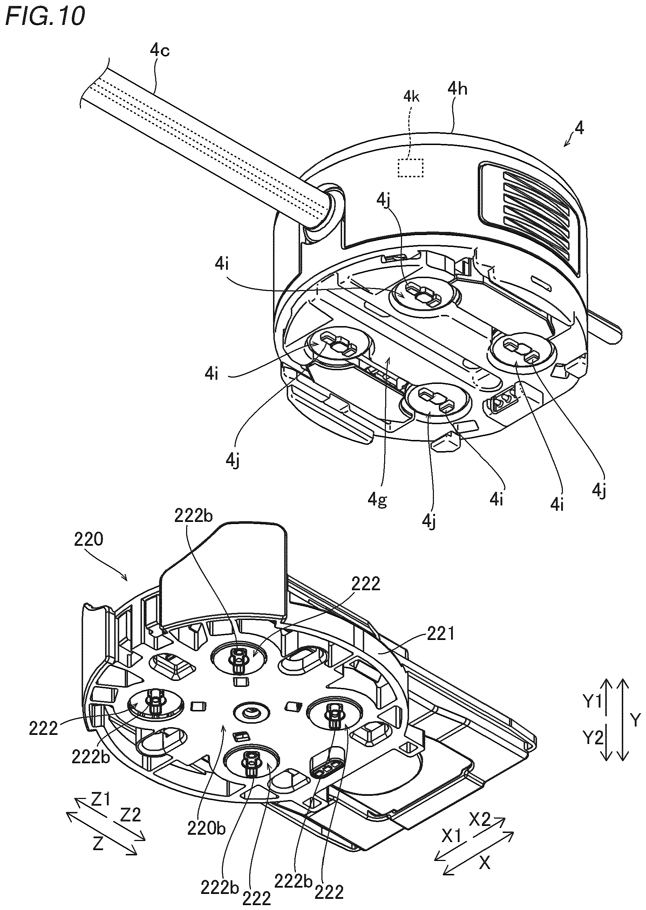

As shown in FIG. 10, the adapter 220 is attached to a connector 4g located on the Y2-direction side of the surgical instrument 4. The connector 4g is arranged in a housing 4h and is attached to the robot arm 60 via the adapter 220. The servomotors M2 of the robot arm 60 are attached to a connector 220b located on the Y2-direction side of the adapter 220. As shown in FIG. 9, the surgical instrument 4 is attached to the connector 220a located on the Y1-direction side of the adapter 220. The adapter 220 is attached to a connector 76 located on the Y1-direction side of the servomotors M2.

As shown in FIG. 10, the surgical instrument 4 includes a storage 4k that stores information about the surgical instrument 4. The information about the surgical instrument 4 is information indicating the type of surgical instrument 4 such as the endoscope 6 or the pair of forceps 4b, for example.

As shown in FIG. 9, the robot arm 60 is covered with the drape 210 for use in a clean area. In the operating room, a cleaning operation is performed to prevent a surgically incised area and medical equipment from being contaminated with pathogens and foreign substances, for example. In this cleaning operation, the clean area and a contaminated area that is an area other than the clean area are set. The surgical site is placed in the clean area. Members of a surgical team including the operator must ensure that only sterile objects are placed in the clean area during surgery, and that an object placed in the contaminated area is sterilized when the object is moved to the clean area. Similarly, when the members of the surgical team including the operator place their hands in the contaminated area, they must sterilize their hands before making direct contact with objects located in the clean area. An instrument used in the clean area is sterilized or covered with the sterile drape 210.

The drape 210 includes a main body 211 that covers the robot arm 60, and a mount 212 that is sandwiched between the servomotors M2 and the adapter 220. The main body 211 is made of a flexible film member formed into a film shape. The flexible film member is made of a resin material such as thermoplastic polyurethane or polyethylene. An opening is formed in the main body 211 such that the servomotors M2 of the robot arm 60 and the adapter 220 can engage with each other. The mount 212 is arranged on the main body 211. The mount 212 is made of a resin molded member. The resin molded member is made of a resin material such as polyethylene terephthalate. The mount 212 is harder than the main body 211. The mount 212 includes an opening such that the servomotors M2 and the adapter 220 can engage with each other. The mount 212 may include an opening to correspond to a portion in which each servomotor M2 and the adapter 220 engage with each other. The mount 212 may include a plurality of openings to correspond to a plurality of portions in which the servomotors M2 and the adapter 220 engage with each other.

As shown in FIG. 9, the adapter 220 includes an adapter main body 221 and a plurality of drive transmitters 222 rotatably held about a rotation axis extending in a Y direction in the adapter main body 221. The plurality of drive transmitters 222 are arranged in the adapter main body 221 so as to be rotatable about the rotation axis. The plurality of drive transmitters 222 are arranged so as to correspond to a plurality of driven members 4i of the surgical instrument 4 shown in FIG. 10. The drive transmitters 222 transmit a driving force from the robot arm 60 to the driven members 4i of the surgical instrument 4. The drive transmitters 222 include fitting recesses 222a into which fitting protrusions 4j of the driven members 4i of the surgical instrument 4 are fitted. The fitting recesses 222a are recessed from Y1-direction side surfaces of the drive transmitters 222 toward the Y2-direction side.

As shown in FIG. 10, the drive transmitters 222 include fitting recesses 222b into which fitting protrusions 75a of the servomotors M2 are fitted. The fitting recesses 222b are recessed from Y2-direction side surfaces of the drive transmitters 222 toward the Y1-direction side.

### Remote Control Apparatus

As shown in FIG. 1, the remote control apparatus 2 is arranged inside or outside the operating room, for example. The remote control apparatus 2 includes an operation unit 120 including arms 121 and an operation handle 21, foot pedals 22, a touch panel 23, a monitor 24, a support arm 25, and a support bar 26. The operation unit 120 includes an operation handle for the operator such as a doctor to input a command.

The operation handle 21 is a handle to operate the surgical instrument 4. The operation handle 21 receives an operation amount for the surgical instrument 4. The operation handle 21 includes an operation handle 21L located on the left side as viewed from the operator such as a doctor and operated by the left hand of the operator, and an operation handle 21R located on the right side and operated by the right hand of the operator.

As shown in FIG. 1, the monitor 24 is a scope-type display that displays an image captured by the endoscope 6. The support arm 25 supports the monitor 24 so as to align the height of the monitor 24 with the height of the face of the operator such as a doctor. The touch panel 23 is arranged on the support bar 26. When the head of the operator is detected by a sensor provided in the vicinity of the monitor 24, the surgical robot 1 can be operated by the remote control apparatus 2. The operator operates the operation handle 21 and the foot pedals 22 while visually recognizing an affected area on the monitor 24. Thus, a command is input to the remote control apparatus 2. The command input to the remote control apparatus 2 is transmitted to the surgical robot 1.

### Configuration of Control System

As shown in FIG. 11, the robotic surgical system 100 includes a control device 130, an arm controller 31a, a positioner controller 31b, and operation controllers 110.

In the present embodiment, the control device 130 is accommodated in the medical cart 3 to communicate with the arm controller 31a and the positioner controller 31b, and controls the entire robotic surgical system 100. Specifically, the control device 130 communicates with and controls the arm controller 31a, the positioner controller 31b, and the operation controllers 110. The control device 130 is connected to the arm controller 31a, the positioner controller 31b, and the operation controllers 110 through a LAN, for example. The control device 130, the arm controller 31a, and the positioner controller 31b are placed inside the medical cart 3.

The arm controller 31a is arranged for each of the plurality of robot arms 60. That is, the same number of arm controllers 31a as the plurality of robot arms 60 are placed inside the medical cart 3.

As shown in FIG. 11, the input 33 is connected to the control device 130 through a LAN, for example. The status indicator 51, the arm status indicator 52, the operation handle 34, the throttle 34a, the joystick 34b, the stabilizer 34c, and the electric cylinder 34d are connected to the positioner controller 31b via a wire line 145 by means of a communication network that allows information to be shared with each other by using serial communication. Although FIG. 11 shows that the status indicator 51, the arm status indicator 52, etc. are all connected to one wire line 145, in reality, the wire line 145 is arranged for each of the status indicator 51, the arm status indicator 52, the operation handle 34, the throttle 34a, the joystick 34b, the stabilizer 34c, and the electric cylinder 34d.

As shown in FIG. 12, the arm portion 61 includes a plurality of servomotors M1, encoders E1, and speed reducers so as to correspond to a plurality of joints 64. The encoders E1 detect rotation angles of the servomotors M1. The speed reducers slow down rotation of the servomotors M1 to increase the torques. The servomotors M1 are examples of a drive. The encoders E1 are examples of a detector.

Inside the medical cart 3, servo controllers C1 that control the servomotors M1 of the robot arm 60 are provided adjacent to the arm controller 31a. The encoders E1 that detect the rotation angles of the servomotors M1 are electrically connected to the servo controllers C1.

As shown in FIG. 12, the servomotors M2 to rotate the driven members 4i provided in the driven unit 4a of the surgical instrument 4, encoders E2, and speed reducers are arranged in the second link 73. The encoders E2 detect rotation angles of the servomotors M2. The speed reducers slow down rotation of the servomotors M2 to increase the torques. In the medical cart 3, servo controllers C2 are provided to control the servomotors M2 to drive the surgical instrument 4. The encoders E2 that detect the rotation angles of the servomotors M2 are electrically connected to the servo controllers C2. A plurality of servomotors M2, a plurality of encoders E2, and a plurality of servo controllers C2 are arranged. The servomotors M2 are examples of a drive. The encoders E2 are examples of a detector.

As shown in FIG. 12, the translation mechanism 70 includes the servomotor M3 to translationally move the surgical instrument 4, an encoder E3, and a speed reducer. The encoder E3 detects a rotation angle of the servomotor M3. The speed reducer slows down rotation of the servomotor M3 to increase the torque. In the medical cart 3, a servo controller C3 is provided to control the servomotor M3 to translationally move the surgical instrument 4. The encoder E3 that detects the rotation angle of the servomotor M3 is electrically connected to the servo controller C3. The servomotor M3 is an example of a drive. The encoder E3 is an example of a detector.

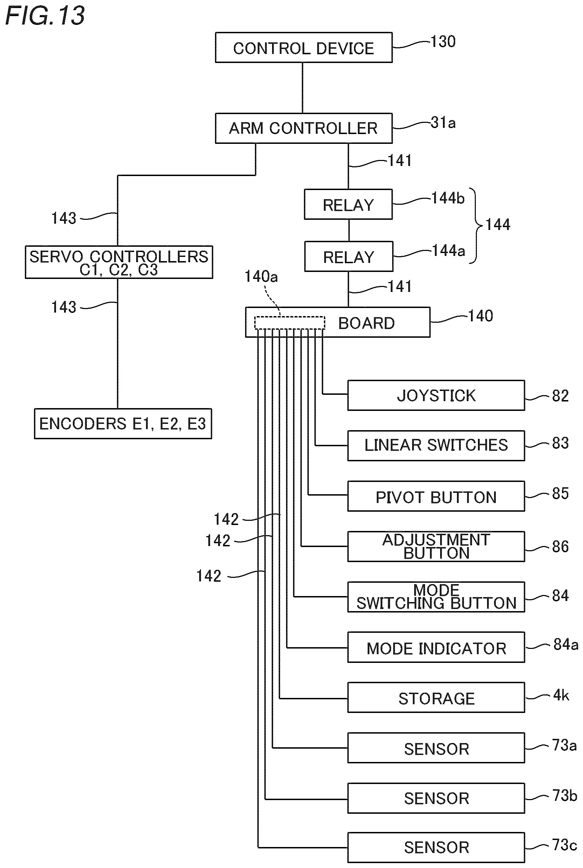

As shown in FIG. 13, the control device 130 controls the robot arm 60 based on an operation received by the arm operation unit 80. For example, the control device 130 controls the robot arm 60 based on an operation received by the joystick 82 of the arm operation unit 80. Specifically, the arm controller 31a outputs an input signal input from the joystick 82 to the control device 130. The control device 130 generates position commands based on the received input signal and the rotation angles detected by the encoders E1, and outputs the position commands to the servo controllers C1 via the arm controller 31a. The servo controllers C1 generate current commands based on the position commands input from the arm controller 31a and the rotation angles detected by the encoders E1, and output the current commands to the servomotors M1. Thus, the robot arm 60 is moved according to an operation command input to the joystick 82.

The control device 130 controls the robot arm 60 based on an input signal from either linear switch 83 of the arm operation unit 80. Specifically, the arm controller 31a outputs the input signal input from the linear switch 83 to the control device 130. The control device 130 generates a position command(s) based on the received input signal and the rotation angle(s) detected by the encoders E1 or the encoder E3, and outputs the position command(s) to the servo controllers C1 or the servo controller C3 via the arm controller 31a. The servo controllers C1 or the servo controller C3 generates a current command(s) based on the position command(s) input from the arm controller 31a and the rotation angle(s) detected by the encoders E1 or the encoder E3, and outputs the current command(s) to the servomotors M1 or the servomotor M3. Thus, the robot arm 60 is moved according to an operation command input to the linear switch 83.

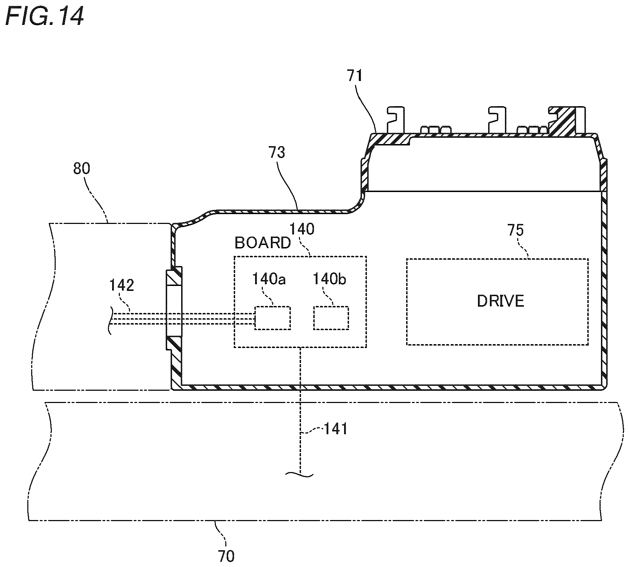

In the present embodiment, as shown in FIG. 14, the robot arm 60 includes a board 140 to which signals received by the arm operation unit 80 are input. The arm controller 31a is connected to the board 140 by serial communication via a first wire line 141. A connector 140a, an IC 140b, etc. are arranged on the board 140. The first wire line 141 includes one transmission path. The arm controller 31a is connected to the board 140 by means of a communication network that allows information to be shared between the arm controller 31a and the board 140 by using serial communication. The first wire line 141 is an example of a wire line.

In the present embodiment, as shown in FIG. 14, the board 140 is placed in the second link 73. The arm operation unit 80 is connected to the second link 73. A plurality of harnesses 142 extending from the arm operation unit 80 are connected to the connector 140a of the board 140. The first wire line 141 including flexible printed wiring extends from the board 140 into the translation mechanism 70.

In the present embodiment, as shown in FIG. 13, the arm controller 31a is connected to the board 140 by serial communication via the first wire line 141 separately from communication paths between the arm controller 31a and the encoders E1, E2, and E3 that detect the movement amounts of the servomotors M1, M2, and M3, respectively. Specifically, the encoders E1, E2, and E3 are connected to the arm controller 31a by serial communication via a second wire line 143. Furthermore, a bus connection is established between the encoders E1, E2, and E3. Moreover, the encoders E1, E2, and E3 are connected to the arm controller 31a via the servo controllers C1, C2, and C3, respectively.

In the present embodiment, as shown in FIG. 2, the arm controller 31a is connected to the board 140 by serial communication via the first wire line 141 through the inside of the robot arm 60 and the outside of the positioner 40. Specifically, the board 140 and the arm controller 31a are connected to each other by the first wire line 141 through the inside of the robot arm 60, the inside of the arm base 50, and the inside of a tube member 44 arranged outside the positioner 40. The tube member 44 is a tube in which a power line etc. are placed.

In the present embodiment, as shown in FIG. 2, the arm controller 31a is connected to the board 140 by serial communication through a relay 144. The relay 144 includes a relay 144a and a relay 144b. The relay 144a is placed on the first link 72, and the board 140 and the relay 144a are connected to each other by the first wire line 141 including the flexible printed wiring. The relay 144b is placed between the robot arm 60 and the arm base 50, and the relay 144a, the relay 144b, and the arm controller 31a are connected to each other through the arm portion 61, the arm base 50, and the inside of the tube member 44 by the first wire line 141 including cable wiring. Specifically, the relay 144a and the relay 144b are connectors. The flexible printed wiring extending from the board 140 and the cable wiring extending from the relay 144b are connected at the relay 144a. Furthermore, the cable wiring extending from the relay 144a and the cable wiring extending from the arm controller 31a are connected at the relay 144b. The relay 144a and the relay 144b may be relay boards equipped with ICs, for example. Furthermore, the relay 144a, the relay 144b, and the arm controller 31a may be connected to each other by flexible printed wiring. Moreover, the relay 144 may be arranged inside the medical cart 3. The flexible printed wiring refers to a flexible printed circuit (FPC). The cable wiring is a wiring structure other than flexible printed wiring, and refers, for example, to a cable covered with an outer jacket made of polyvinyl chloride resin, polyethylene resin, ethylene tetrafluoroethylene (ETFE) resin, or the like. The cable wiring is sometimes referred to as a robot cable. A flexible flat cable (FFC) may be used as the flexible printed wiring.

In the present embodiment, as shown in FIG. 13, at least one of a signal received by the joystick 82, signals received by the linear switches 83, a signal received by the pivot button 85, or a signal received by the adjustment button 86 is input to the board 140. Specifically, all signals received by the joystick 82, the linear switches 83, the pivot button 85, and the adjustment button 86 are input to the board 140. The joystick 82, the linear switches 83, the pivot button 85, and the adjustment button 86 are connected to the connector 140a of the board 140 by the harnesses 142. An analog signal is transmitted from the joystick 82. Digital signals are transmitted from the linear switches 83, the pivot button 85, and the adjustment button 86.

In the present embodiment, a signal received by the mode switching button 84 is input to the board 140. The signal is output from the board 140 to the mode indicator 84a. A digital signal is transmitted from the mode switching button 84.

In the present embodiment, at least one of information about the surgical instrument 4 from the storage 4k arranged in the surgical instrument 4, information about whether or not the surgical instrument 4 is attached to the robot arm 60, or information about whether or not the adapter 220 for attaching the surgical instrument 4 is attached to the robot arm 60 is input to the board 140. Specifically, the information about the surgical instrument 4 from the storage 4k, the information about whether or not the surgical instrument 4 is attached, and the information about whether or not the adapter 220 is attached are all input to the board 140. As shown in FIG. 9, whether or not the surgical instrument 4 is attached to the robot arm 60 is detected by a sensor 73a arranged on the second link 73. Whether or not the adapter 220 is attached to the robot arm 60 is detected by the sensor 73b arranged on the second link 73. Whether or not the adapter 220 is attached to the robot arm 60 is detected by a sensor 73b arranged on the second link 73.

Information about whether or not the fitting protrusions 4j of the driven members 4i of the surgical instrument 4 are fitted into the fitting recesses 222a of the drive transmitter 222 of the adapter 220 is input to the board 140. Whether or not the fitting protrusions 4j are fitted into the fitting recesses 222a is detected by a sensor 73c arranged on the second link 73.

As shown in FIG. 11, the positioner controller 31b controls the positioner 40 and the medical cart 3. Servomotors SM, encoders EN, and speed reducers are provided in the positioner 40 so as to correspond to a plurality of joints 43 of the positioner 40. Servo controllers SC are provided in the medical cart 3 to control the servomotors SM of the positioner 40. Servomotors SM that drive a plurality of front wheels of the medical cart 3, encoders EN, speed reducers, servo controllers SC, and brakes are provided in the medical cart 3.

As shown in FIG. 11, the operation controllers 110 are arranged in a main body of the remote control apparatus 2. The operation controllers 110 control the operation unit 120. The operation controllers 110 are provided for the operation unit 120L and the operation unit 120R, respectively. Servomotors SM, encoders EN, and speed reducers are provided in the operation unit 120 so as to correspond to a plurality of joints of the operation unit 120. Servo controllers SC that control the servomotors SM of the operation unit 120 are provided adjacent to the operation controllers 110 in the main body of the remote control apparatus 2.

### Advantages of Present Embodiment

The board 140 to which a signal received by the arm operation unit 80 is input is connected to the arm controller 31a by serial communication via the first wire line 141. Accordingly, even when the joystick 82, an operation button, etc. are arranged on the arm operation unit 80, the harnesses 142 extending from the joystick 82, the operation button, etc. are connected to the board 140, while the board 140 is connected to the arm controller 31a by serial communication. Consequently, the number of wire lines can be reduced as compared with a case in which the joystick 82, the operation button, etc. are each connected to the arm controller 31a.

The arm controller 31a is connected to the board 140 by serial communication via the first wire line 141 separately from the communication paths between the arm controller 31a and the encoders E1, E2, and E3 that detect the movement amounts of the servomotors M1, M2, and M3, respectively. Accordingly, interference between a signal between the arm operation unit 80 and the arm controller 31a and signals between the encoders E1, E2, and E3 and the arm controller 31a can be reduced or prevented.

The encoders E1, E2, and E3 are connected to the arm controller 31a by serial communication via the second wire line 143. Accordingly, even when a plurality of encoders E1, E2, and E3 are arranged, the number of wire lines extending to the arm controller 31a can be reduced while interference between the signal from the arm operation unit 80 and the signal from each encoder is reduced or prevented.

The arm controller 31a is connected to the board 140 by serial communication through the inside of the robot arm 60. Accordingly, the board 140 and the arm controller 31a are connected to each other by the first wire line 141, and the number of wire lines is reduced. Thus, an increase in the size of the robot arm 60 can be reduced or prevented.

The arm controller 31a is placed inside the medical cart 3, and the arm controller 31a is connected to the board 140 by serial communication via the first wire line 141 through the inside of the robot arm 60 and the outside of the positioner 40. Accordingly, the number of wire lines through the inside of the robot arm 60 and the outside of the positioner 40 can be reduced.

The control device 130 is accommodated in the medical cart 3 to communicate with the arm controller 31a. Accordingly, unlike a case in which the control device 130 is provided outside the medical cart 3, the complexity of the configuration of the robotic surgical system 100 can be reduced or prevented.

The arm controller 31a is connected to the board 140 by serial communication through the relay 144. Accordingly, the relay 144 is arranged between portions to be separated from each other such that the portions to be separated from each other can be separated integrally with the wire line. Consequently, work such as reconnecting another wire line can be easily performed in the robotic surgical system 100, and thus the workload can be reduced. Furthermore, the type of wire line by which a serial communication connection is established through the relay 144 can be changed. For example, the cable wiring and the flexible printed wiring are connected to each other through the relay 144 such that the flexible printed wiring can be arranged in a movable portion.

The board 140 and the relay 144a are connected to each other by the first wire line 141 including the flexible printed wiring. Accordingly, the first wire line 141 including the flexible printed wiring is arranged between the second link 73 and the translation mechanism 70, and thus obstruction of movement of the second link 73 that moves relatively linearly along the Z direction and the translation mechanism 70 by the first wire line 141 can be reduced or prevented.

At least one of the signal received by the joystick 82, the signals received by the linear switches 83, the signal received by the pivot button 85, or the signal received by the adjustment button 86 is input to the board 140. Accordingly, when at least one of the joystick 82, the linear switches 83, the pivot button 85, or the adjustment button 86 is arranged on the arm operation unit 80, the number of wire lines extending to the arm controller 31a can be reduced. Furthermore, when more than one of the joystick 82, the linear switches 83, the pivot buttons 85, and the adjustment buttons 86 are arranged on arm operation unit 80, the number of wire lines extending from the arm operation unit 80 is increased, and thus it is particularly effective to reduce the number of wire lines extending to the arm controller 31a by connecting the arm controller 31a to the board 140 by serial communication.

The signal received by the mode switching button 84 is input to the board 140, and is output from the board 140 to the mode indicator 84a. Accordingly, when the mode indicator 84a is arranged on the arm operation unit 80 and a signal is output from the board 140 to the mode indicator 84a, the number of wire lines extending to the arm controller 31a can be reduced.

At least one of the information about the surgical instrument 4 from the storage 4k, the information about whether or not the surgical instrument 4 is attached to the robot arm 60, or the information about whether or not the adapter 220 for attaching the surgical instrument 4 is attached to the robot arm 60 is input to the board 140. Accordingly, when at least one of the information about the surgical instrument 4, the information about whether or not the surgical instrument 4 is attached, or the information about whether or not the adapter 220 is attached is input to the board 140, the number of wire lines extending to the arm controller 31a can be reduced.

The board 140 is placed in the second link 73. Accordingly, a distance between the arm operation unit 80 and the board 140 is relatively small, and thus the influence of noise on the signal input from the arm operation unit 80 to the board 140 can be reduced.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the example in which the arm controller 31a is placed in the medical cart 3 has been shown in the aforementioned embodiment, the present disclosure is not limited to this. For example, the arm controller 31a may be placed in a portion other than the medical cart 3, such as the robot arm 60.

While the example in which the arm operation unit 80 is attached to the second link 73 has been shown in the aforementioned embodiment, the present disclosure is not limited to this. For example, the arm operation unit 80 may be attached to a portion of the robot arm 60 other than the second link 73.

While the example in which the board 140 is placed in the second link 73 has been shown in the aforementioned embodiment, the present disclosure is not limited to this. For example, the board 140 may be placed in the arm operation unit 80.

While the example in which the encoders E1, E2, and E3 are connected to the arm controller 31a by serial communication via the second wire line 143 has been shown in the aforementioned embodiment, the present disclosure is not limited to this. For example, a communication method between the encoders E1, E2, and E3 and the arm controller 31a may be other than serial communication.

While the example in which the control device 130 is accommodated in the medical cart 3 has been shown in the aforementioned embodiment, the present disclosure is not limited to this. For example, the control device 130 may be provided outside the medical cart 3.

While the example in which the arm controller 31a is connected to the board 140 by serial communication through the relay 144 has been shown in the aforementioned embodiment, the present disclosure is not limited to this. For example, the arm controller 31a and the board 140 may be directly connected to each other without using the relay 144.

While the example in which all signals received by the joystick 82, the linear switches 83, the pivot button 85, and the adjustment button 86 are input to the board 140 has been shown in the aforementioned embodiment, the present disclosure is not limited to this. For example, a signal from any one of the joystick 82, the linear switches 83, the pivot button 85, and the adjustment button 86 or signals from more than one, but not all, of the joystick 82, the linear switches 83, the pivot button 85, and the adjustment button 86 may be input to the board 140.

While the example in which four robot arms 60 are provided has been shown in the aforementioned embodiment, the present disclosure is not limited to this. In the present disclosure, the number of robot arms 60 may be any number as long as at least one robot arm 60 is provided.

While the example in which each of the arm portion 61 and the positioner 40 includes a 7-axis articulated robot has been shown in the aforementioned embodiment, the present disclosure is not limited to this. For example, each of the arm portion 61 and the positioner 40 may include an articulated robot having an axis configuration other than the 7-axis articulated robot. The axis configuration other than the 7-axis articulated robot includes six axes or eight axes, for example.

While the example in which the surgical robot 1 includes the medical cart 3, the positioner 40, and the arm base 50 has been shown in the aforementioned embodiment, the present disclosure is not limited to this. For example, the surgical robot 1 may not include the medical cart 3, the positioner 40, or the arm base 50, but may include only the robot arms 60.

### Aspects

It will be appreciated by those skilled in the art that the exemplary embodiments described above are specific examples of the following aspects.

### (Item 1)

A robotic surgical system comprising:
a robot arm to which a surgical instrument is attached;
an arm operation unit attached to the robot arm to operate the robot arm;
a board provided in the robot arm or the arm operation unit and to which a signal received by the arm operation unit is input; and
a controller; wherein
the controller is connected to the board by serial communication via a first wire line.

### (Item 2)

The robotic surgical system according to item 1, wherein
the robot arm includes a joint and a drive provided in the joint; and
the controller is connected to the board by serial communication via the first wire line separately from a communication path between the controller and a detector operable to detect a movement amount of the drive.

### (Item 3)

The robotic surgical system according to item 2, wherein the detector is connected to the controller by serial communication via a second wire line.

### (Item 4)

The robotic surgical system according to any one of items 1 to 3, wherein the controller is connected to the board by serial communication through an inside of the robot arm.

### (Item 5)

The robotic surgical system according to item 4, further comprising:
an arm base to which the robot arm is attached;
a positioner to adjust a position of the arm base; and
a medical cart to move the positioner; wherein
the controller is placed inside the medical cart; and the controller is connected to the board by serial communication via the first wire line through the inside of the robot arm and an outside of the positioner.

### (Item 6)

The robotic surgical system according to item 5, further comprising:
a control device accommodated in the medical cart to communicate with the controller and configured or programmed to control an entirety of the robotic surgical system.

### (Item 7)

The robotic surgical system according to any one of items 1 to 6, wherein the controller is connected to the board by serial communication through a relay.

### (Item 8)

The robotic surgical system according to item 7, wherein the relay is connected to at least one of the controller or the board by serial communication via the first wire line including flexible printed wiring.

### (Item 9)

The robotic surgical system according to item 8, wherein
the robot arm includes:
   an arm portion;
   a first link placed at a distal end of the arm portion;
   a second link to which the arm operation unit is attached; and
   a translation mechanism to translate the second link with respect to the first link;
the relay includes a first relay placed on the first link; and
the board is connected to the first relay by serial communication via the first wire line including the flexible printed wiring.

### (Item 10)

The robotic surgical system according to item 9, further comprising:
an arm base to which the robot arm is attached; wherein
the relay includes a second relay placed between the robot arm and the arm base; and
the second relay is connected to the first relay and the controller by serial communication via the first wire line including cable wiring.

### (Item 11)

The robotic surgical system according to any one of items 1 to 10, wherein
the arm operation unit includes at least one of:
   a joystick to control movement of the surgical instrument by the robot arm;
   a linear switch to control movement of the surgical instrument by the robot arm in a direction along a longitudinal direction of the surgical instrument;
   a pivot button to set a pivot position that serves as a fulcrum for movement of the surgical instrument attached to the robot arm; or
   an adjustment button to optimize a position of the robot arm; and
at least one of a signal received by the joystick, a signal received by the linear switch, a signal received by the pivot button, or a signal received by the adjustment button is input to the board.

### (Item 12)

The robotic surgical system according to any one of items 1 to 11, wherein
the arm operation unit includes:
   a mode switching button to switch between a mode for translationally moving the surgical instrument and a mode for rotationally moving the surgical instrument; and
   a mode indicator to indicate a selected mode; and
a signal received by the mode switching button is input to the board, and is output from the board to the mode indicator.

### (Item 13)

The robotic surgical system according to any one of items 1 to 12, wherein
the surgical instrument includes a storage to store information about the surgical instrument;
the surgical instrument is attached to the robot arm via an adapter; and
at least one of the information about the surgical instrument from the storage, information about whether or not the surgical instrument is attached to the robot arm, or information about whether or not the adapter for attaching the surgical instrument is attached to the robot arm is input to the board.

### (Item 14)

The robotic surgical system according to any one of items 1 to 13, wherein
the robot arm includes:
   an arm portion;
   a first link placed at a distal end of the arm portion;
   a second link to which the arm operation unit is attached; and
   a translation mechanism to translate the second link with respect to the first link; and
the board is placed in the second link.

### (Item 15)

A surgical robot comprising:
a robot arm to which a surgical instrument is attached;
an arm operation unit attached to the robot arm to operate the robot arm;
a board provided in the robot arm or the arm operation unit and to which a signal received by the arm operation unit is input; and
a controller; wherein
the controller is connected to the board by serial communication via a wire line.

## Claims

1. A robotic surgical system comprising:
a robot arm to which a surgical instrument is attached;
an arm operation unit attached to the robot arm to operate the robot arm;
a board provided in the robot arm or the arm operation unit and to which a signal received by the arm operation unit is input; and
a controller; wherein
the controller is connected to the board by serial communication via a first wire line.

2. The robotic surgical system according to claim 1, wherein
the robot arm includes a joint and a drive provided in the joint; and
the controller is connected to the board by serial communication via the first wire line separately from a communication path between the controller and a detector operable to detect a movement amount of the drive.

3. The robotic surgical system according to claim 2, wherein the detector is connected to the controller by serial communication via a second wire line.

4. The robotic surgical system according to claim 1, wherein the controller is connected to the board by serial communication through an inside of the robot arm.

5. The robotic surgical system according to claim 4, further comprising:
an arm base to which the robot arm is attached;
a positioner to adjust a position of the arm base; and
a medical cart to move the positioner; wherein
the controller is placed inside the medical cart; and
the controller is connected to the board by serial communication via the first wire line through the inside of the robot arm and an outside of the positioner.

6. The robotic surgical system according to claim 5, further comprising:
a control device accommodated in the medical cart to communicate with the controller and configured or programmed to control an entirety of the robotic surgical system.

7. The robotic surgical system according to claim 1, wherein the controller is connected to the board by serial communication through a relay.

8. The robotic surgical system according to claim 7, wherein the relay is connected to at least one of the controller or the board by serial communication via the first wire line including flexible printed wiring.

9. The robotic surgical system according to claim 8, wherein
the robot arm includes:
an arm portion;
a first link placed at a distal end of the arm portion;
a second link to which the arm operation unit is attached; and
a translation mechanism to translate the second link with respect to the first link;
the relay includes a first relay placed on the first link; and
the board is connected to the first relay by serial communication via the first wire line including the flexible printed wiring.

10. The robotic surgical system according to claim 9, further comprising:
an arm base to which the robot arm is attached; wherein
the relay includes a second relay placed between the robot arm and the arm base; and
the second relay is connected to the first relay and the controller by serial communication via the first wire line including cable wiring.

11. The robotic surgical system according to claim 1, wherein
the arm operation unit includes at least one of:
a joystick to control movement of the surgical instrument by the robot arm;
a linear switch to control movement of the surgical instrument by the robot arm in a direction along a longitudinal direction of the surgical instrument;
a pivot button to set a pivot position that serves as a fulcrum for movement of the surgical instrument attached to the robot arm; or
an adjustment button to optimize a position of the robot arm; and
at least one of a signal received by the joystick, a signal received by the linear switch, a signal received by the pivot button, or a signal received by the adjustment button is input to the board.

12. The robotic surgical system according to claim 1, wherein
the arm operation unit includes:
a mode switching button to switch between a mode for translationally moving the surgical instrument and a mode for rotationally moving the surgical instrument; and
a mode indicator to indicate a selected mode; and
a signal received by the mode switching button is input to the board, and is output from the board to the mode indicator.

13. The robotic surgical system according to claim 1, wherein
the surgical instrument includes a storage to store information about the surgical instrument;
the surgical instrument is attached to the robot arm via an adapter; and
at least one of the information about the surgical instrument from the storage, information about whether or not the surgical instrument is attached to the robot arm, or information about whether or not the adapter for attaching the surgical instrument is attached to the robot arm is input to the board.

14. The robotic surgical system according to claim 1, wherein
the robot arm includes:
an arm portion;
a first link placed at a distal end of the arm portion;
a second link to which the arm operation unit is attached; and
a translation mechanism to translate the second link with respect to the first link; and
the board is placed in the second link.

15. A surgical robot comprising:
a robot arm to which a surgical instrument is attached;
an arm operation unit attached to the robot arm to operate the robot arm;
a board provided in the robot arm or the arm operation unit and to which a signal received by the arm operation unit is input; and
a controller; wherein
the controller is connected to the board by serial communication via a wire line.
